# EUROPEAN PATENT APPLICATION

(11) **EP 4 300 071 A1**
(43) Date of publication of application: **03.01.2024**
(21) Application number: 23178582.5
(22) Date of filing: 12.06.2023
(51) Int. Cl.: G01N 1/22, G01N 21/33, G01N 33/00

(54) **METHOD OF MONITORING ODOR-CAUSING SUBSTANCE IN FLUE GAS**

(30) Priority: 28.06.2022 KR 20220079206
(71) Applicant: SK Innovation Co., Ltd., Seoul 03188 (KR)
(72) Inventor: JANG, Yoon Hyuk, 34124 Daejeon (KR); KIM, Tae Wan, 34124 Daejeon (KR); SONG, Jae Yang, 34124 Daejeon (KR)
(74) Representative: Frick, Robert

(57) **Abstract**

The invention relates to a method of monitoring the presence and preferably the type and/or amount of one or more odor-causing substances in flue gas, which comprises: providing flue gas containing one or more odor-causing substances; introducing the flue gas into an odor removal unit to produce a gas-phase stream; dissolving a portion of the gas-phase stream in a solvent to produce a liquid-phase stream; and measuring an absorbance of the liquid-phase stream to detect and preferably identify and/or quantify the one or more odor-causing substances.

## Description

### BACKGROUND OF THE DISCLOSURE

### 1. Field of the Disclosure

The present disclosure relates to a method of monitoring odor-causing substances in flue gas. More particularly, the present disclosure relates to a method of controlling a removal process of odor-causing substances in flue gas by analyzing types and concentrations of the odor-causing substances using the absorbance thereof.

### 2. Description of the Related Art

With the rapid expansion of industrial facilities, ever-increasing environmental pollution problems have become a critical social issue. Among such problems, odorous gases, along with environmental noise, are becoming the most common public complaint. Hence, odor regulation is becoming increasingly strict, and demand for deodorization technology capable of removing odorous gases is thus growing.

Odors typically refer to unpleasant and repulsive smells caused when irritating substances, such as hydrogen sulfide, mercaptans, amines, and the like, stimulate the human sense of smell. In addition, complex odors refer to unpleasant and repulsive smells caused when two or more types of these irritating substances are combined and stimulate the human sense of smell.

In Korea, for example, odors are regulated by the current Korean Odor Prevention Law, which defines odors as 22 types of designated odorous substances and complex odors. The designated odorous substances are to be measured using instrumental analysis methods, and the complex odors are measured using the air dilution sensory method based on the Official Test Method enacted by the Korean Ministry of Environment according to Article 6, Paragraph (1), Item 4 of the Korean Environmental Testing and Inspection ACT. Standards for permissible emissions of complex odors under the current Korean law are shown in Table 1 below.

**[Table 1]**

| Classification | Standards for permissible emissions (dilution factor) | | Strict standards for permissible emissions (dilution factor) | |
|---|---|---|---|---|
| | Industrial area | Other areas | Industrial area | Other areas |
| Outlet | 1000 or lower | 500 or lower | 500 to 1000 | 300 to 500 |
| Property boundary | 20 or lower | 15 or lower | 15 to 20 | 10 to 15 |

Here, the dilution factor refers to a factor obtained by diluting the collected sample with odorless air in a stepwise manner until no odor is detected. As can be seen here, the evaluators had no choice but to evaluate the level of complex odors subjectively by smelling, unlike in the case of designated odorous substances. Real-time monitoring of complex odors based on more objective criteria is necessary for businesses not to exceed the regulated standards for permissible emissions. In addition, even though there are methods of using gas chromatography equipment and the like as conventional methods of determining odor-causing substances, analysis preparation for such methods and the analysis itself require a lot of time and manpower, not to mention that such equipment is also expensive.

Furthermore, although odor-measuring devices are used to determine odors in some cases, such devices have poor accuracy, and real-time measurement is difficult due to inconvenient measurement processes. Moreover, there is a problem in terms of maintenance because sensor replacement and calibration are periodically required.

### SUMMARY OF THE INVENTION

Against this background, the invention provides a method of monitoring the presence and preferably the type and/or amount of one or more odor-causing substances in real time in flue gas by measuring the absorbance of the odor-causing substances.

The method comprises: providing flue gas containing one or more odor-causing substances; introducing the flue gas into an odor removal unit to produce a gas-phase stream; dissolving a portion of the gas-phase stream in a solvent to produce a liquid-phase stream; and measuring an absorbance of the liquid-phase stream to detect and preferably identify and/or quantify the one or more odor-causing substances.

According to one embodiment, the odor-causing substances comprise one or more of ethanol, benzene, trimethyl benzene, benzaldehyde, benzoic acid, toluene, phenol, styrene, α-methylstyrene, indole, pyridine, skatole, methylcyclohexane, xylene, o-cresol, m-cresol, methyl ethyl ketone, methyl isobutyl ketone, ammonia, methylamine, ethylamine, dimethylamine, trimethylamine, acetaldehyde, propionaldehyde, butyraldehyde, n-valeraldehyde, i-valeraldehyde, ethyl acetate, butyl acetate, propionic acid, n-butyric acid, n-valeric acid, i-valeric acid, i-butyl alcohol, ethyl acrylate, sulfur dioxide, hydrogen sulfide, dimethyl sulfide, dimethyl disulfide, diethyl disulfide, methyl ethyl sulfide, methyl mercaptan, ethyl mercaptan, carbon disulfide, methyl diethanolamine, diisopropylamine, acrolein, chlorine, or fluorine.

In one embodiment, the flue gas is introduced into the bottom of the odor removal unit. The odor removal unit may be a scrubber, a combustion device, an adsorption device, or a unit comprising a combination of such devices.

In one embodiment, the odor removal unit is a scrubber. Water may be supplied to the top of the scrubber.

In one embodimenture, the solvent comprises one or more of water, an aqueous solution, or an organic solvent.

In one embodiment, the aqueous solution is a NaOH solution or H₂SO₄ solution.

In one embodiment, the absorbance is measured using a spectrophotometer or optical sensor.

In one embodiment, the absorbance is measured in a wavelength range of 170 nm to 280 nm.

In one embodiment, the method further comprises: analyzing the odor-causing substances based on the measured absorbance to obtain an analysis result; and controlling operating conditions in the odor removal unit based on the analysis result.

Using a monitoring method provided herein, types and concentrations of odor-causing substances generated from odor removal equipment can be monitored in real time at low costs.

In addition, using the standard absorbance data for each odor-causing substance, not only real-time analysis of the odor-causing substances generated in flue gas but also control of operating conditions in the odor removal equipment according to the analysis results are automatable. As a result, the operation can be optimized, and manpower and costs thus can be additionally reduced.

### BRIEF DESCRIPTION OF THE DRAWINGS

FIGS. 1 to 4 are flowcharts illustrating each process of monitoring odor-causing substances in flue gas according to one embodiment of the present disclosure.
FIG. 5 is a graph depicting absorbance measurement results for each odor-causing substance, according to an example.

### DESCRIPTION OF THE PREFERRED EMBODIMENTS

The invention relates to a method of monitoring the presence and preferably the type and/or amount of one or more odor-causing substances in flue gas.

The method comprises: providing flue gas containing one or more odor-causing substances; introducing the flue gas into an odor removal unit to produce a gas-phase stream; dissolving a portion of the gas-phase stream in a solvent to produce a liquid-phase stream; and measuring an absorbance of the liquid-phase stream to detect and preferably identify and/or quantify the one or more odor-causing substances.

In the present disclosure, "flue gas" refers to gas derived from stationary sources, such as petrochemical plants, power plants, and the like, and mobile sources, such as vehicles, buses, trains, and the like, but is not limited thereto. In addition, any gas containing the odor-causing substances may fall within the target gas to be processed in the present disclosure.

In the present disclosure, "odor-causing substance" refers to a substance that causes the odor described above, and is not particularly limited as long as it is mixed in the air and causes odor. In particular, the odor-causing substance is not construed to be limited to the 22 types of designated odorous substances described above. In one embodiment of the present disclosure, the odor-causing substance may comprise ethanol, benzene, trimethyl benzene, benzaldehyde, benzoic acid, toluene, phenol, styrene, α-methylstyrene, indole, pyridine, skatole, methylcyclohexane, xylene, o-cresol, m-cresol, methyl ethyl ketone, methyl isobutyl ketone, ammonia, methylamine, ethylamine, dimethylamine, trimethylamine, acetaldehyde, propionaldehyde, butyraldehyde, *n*-valeraldehyde, i-valeraldehyde, ethyl acetate, butyl acetate, propionic acid, *n*-butyric acid, *n*-valeric acid, i-valeric acid, i-butyl alcohol, ethyl acrylate, sulfur dioxide, hydrogen sulfide, dimethyl sulfide, dimethyl disulfide, diethyl disulfide, methyl ethyl sulfide, methyl mercaptan, ethyl mercaptan, carbon disulfide, methyl diethanolamine, diisopropylamine, acrolein, chlorine, fluorine, or a combination thereof.

For example, sulfur compounds, such as methyl mercaptan, are characterized by the smell of rotten onions and rotten cabbages, and hydrogen sulfide is characterized by the smell of rotten eggs. In addition, nitrogen compounds, such as ammonia and methyl amine, are characterized by the smell of excrement and rotten fish. Aldehydes, such as acetaldehyde, are characterized by an irritating, sour, and burning smell, and hydrocarbons, such as ethyl acetate and propionic acid, and fatty acids are characterized by the irritating smell of paint thinners. Furthermore, halogens, such as chlorine and fluorine, are characterized by an irritating smell. Such odor-causing substances described above make those who smell the odor unpleasant and disgusted, and longer periods of smelling may accompany dizziness and/or headaches.

In one embodiment, the flue gas comprises the odor-causing substances described above. For example, the flue gas may comprise odor-causing substances at concentrations (ppm v/v) equivalent to or higher than the lower limit regulated by the Korean Ministry of Environment. The minimum detectable concentrations of major chemicals according to the Enforcement Rules of the Korean Clean Air Conservation Act are shown in Table 2 below.

**[Table 2]**

| Chemical Name | Concentration (ppm) (v/v) | Chemical Name | Concentration (ppm) (v/v) |
|---|---|---|---|
| Ammonia | 0.1 | Formaldehyde | 0.02 |
| Methyl mercaptan | 0.0001 | Acrolein | 0.0085 |
| Hydrogen sulfide | 0.0005 | Acrylonitrile | 8.8 |
| Dimethyl sulfide | 0.0001 | Methanol | 0.52 |
| Dimethyl disulfide | 0.0003 | Dimethylamine | 0.033 |
| Trimethylamine | 0.0001 | Methylamine | 0.035 |
| Acetaldehyde | 0.002 | Acetic acid | 0.0057 |
| Propionaldehyde | 0.002 | Benzene | 2.7 |
| n-Butyraldehyde | 0.0003 | Phenol | 0.00028 |
| i-Butyraldehyde | 0.0009 | Carbon disulfide | 0.21 |
| n-Valeraldehyde | 0.0007 | Pyridine | 0.063 |
| i-Valeraldehyde | 0.0002 | Methyl alkyl sulfide | 0.00014 |
| i-Butanol | 0.01 | Carbon tetrachloride | 4.6 |
| Ethyl acetate | 0.3 | Chloroform | 3.8 |
| Methyl isobutyl ketone | 0.2 | Indole | 0.00030 |
| Toluene | 0.9 | Skatole | 0.0000056 |
| Styrene | 0.03 | Ethyl benzene | 0.17 |
| *o*-Xylene | 0.38 | 1,3-Butadiene | 0.23 |
| *m*-Xylene | 0.041 | Diethyl sulfide | 0.000033 |
| *p*-Xylene | 0.058 | Ethanol | 0.094 |
| Propionic acid | 0.002 | Ethyl acrylate | 0.00026 |
| *n*-Butyric acid | 0.00007 | Ethyl mercaptan | 0.0000087 |
| *n*-Valeric acid | 0.0001 | Methyl ethyl ketone | 0.44 |
| i-Valeric acid | 0.00005 | Sulfur dioxide | 0.055 |
| 1,2,4-Trimethyl benzene | 0.12 | Nitrogen dioxide | 0.12 |
| 1,3,5-Trimethyl benzene | 0.17 | Methyl acetate | 1.7 |
| Acetone | 42 | Ethyl acetate | 0.87 |
| Dichloromethane | 160 | i-Butyl acetate | 0.0080 |
| Trichloroethylene | 3.9 | *o*-Cresol | 0.00010 |
| Tetrachloroethylene | 0.77 | *m*-Cresol | 0.000054 |

According to the invention, the flue gas comprising the odor-causing substances is introduced into the odor removal unit. While passing through the odor removal unit, the odor-causing substances in the flue gas may be removed, or the number of the odor-causing substances may be reduced.

The odor removal unit refers to a device, a system, a means, and the like capable of removing the odor-causing substances in the flue gas. The odor removal unit is not particularly limited as long as the unit is an odor removal means having a structure where the flue gas is introduced, and the gas with the reduced number of the odor-causing substances is discharged. For example, the odor removal means may comprise scrubbing, combustion, adsorption, chemical process, cooling condensation, biological process, and the like.

In one embodiment, the odor removal unit is a scrubber, a combustion device, an adsorption device, or a unit comprising a combination of such devices. In another embodiment, the odor removal unit is composed of a plurality of devices arranged in parallel and/or series, where the plurality of devices may be the same or different from each other.

A scrubber reduces the number of odor-causing substances by spraying water (or a combination of water and an absorbent) onto the introduced flue gas to bring the odor-causing substances in the flue gas into contact with water. In addition, an adsorption device reduces the number of odor-causing substances by using the characteristics of the odor-causing substances being adsorbed in between the pores of an adsorbent when flue gas introduced into the device allows the adsorbent present in the device to pass through. Furthermore, a combustion device reduces the number of odor-causing substances by allowing the odor-causing substances in flue gas introduced into the device to undergo oxidation combustion or thermal degradation at high temperatures. When using the scrubber as the odor removal unit, the scrubber may produce a water stream, and the water stream is discharged from the bottom of the scrubber. The water stream may refer to scrubber wastewater comprising odor-causing substances brought into contact with the water supplied to the scrubber.

In one embodiment, the flue gas is introduced into the bottom of the odor removal unit. In the odor removal unit, as the flue gas flows upward from the bottom to the top, the number of the odor-causing substances is reduced. Lastly, the flue gas is discharged from the top of the odor removal unit as a gas-phase stream to be described later.

In one embodiment, the odor removal unit is a scrubber. In this case, water may be supplied to the top of the scrubber from the outside, and the supplied water then makes contact with the flue gas while flowing downward in the scrubber. On the other hand, in the case of performing the monitoring method of the present invention in an environment involving odor-causing substances likely to be lipophilic, an organic solvent, instead of water, may be used in the scrubber.

The odor removal unit produces the gas-phase stream. The gas-phase stream refers to flue gas in which the number of the odor-causing substances is reduced through the odor removal unit. The gas-phase stream is discharged from the top of the odor removal unit.

According to the invention, a portion of the gas-phase stream is dissolved in the solvent to produce a liquid-phase stream, which is used for absorbance measurement of the odor-causing substances as described later. The portion of the gas-phase stream may be dissolved in an extremely small amount with respect to the total amount of the gas-phase stream. The solvent used herein is not particularly limited as long as the solvent does not adversely affect the absorbance measurement to be described later and is capable of dissolving the odor-causing substances well. In one embodiment, the solvent comprises one or more of water, an aqueous solution, or an organic solvent. Here, when using a random combination of the water, the aqueous solution, and the organic solvent as the solvent, the portion of the gas-phase stream is not dissolved in a random mixture of the water, aqueous solution, and organic solvent. Rather, this means each independently dissolving the portion of the gas-phase stream in the water, the aqueous solution, and/or the organic solvent. As the organic solvent, n-pentane, n-hexane, dichloromethane, methanol, ethanol, 2-methylbutane, ethyl acetate, tetrahydrofuran, acetonitrile, dimethyl sulfoxide, and the like may be used. However, the organic solvent is not limited thereto. In addition, the examples of the organic solvent may be used solely or in combination.

Depending on the types of odor-causing substance, the liquid-phase stream may vary in acidity. In one embodiment, the pH of the solvent may be appropriately adjusted to increase the solubility of the liquid-phase stream.

For example, when the odor-causing substances are acidic, an aqueous solution of NaOH may be used as the solvent. In addition, when the odor-causing substances are basic, an aqueous solution of H₂SO₄ may be used as the solvent.

The dissolving of the portion of the gas-phase stream may further comprise removing solids. The gas-phase stream before the dissolving and the produced liquid-phase stream may comprise solids. Such solids may act as a cause of error in the absorbance measurement. Thus, the solids are preferably removed from the liquid-phase stream before the absorbance measurement to reduce errors. A means to remove the solids is not particularly limited, and the solids may be removed by mechanical means such as a filter or centrifugation.

Next, according to the invention the absorbance of the liquid-phase stream produced above is measured. In one embodiment, the absorbance of the liquid-phase stream is measured using a spectrophotometer or optical sensor. The spectrophotometer may be an ultraviolet (UV) spectrophotometer or ultraviolet-visible (UV-Vis) spectrophotometer. The electronic structure of atoms or molecules of the odor-causing substances, as well as the compositions thereof, may be analyzed at peaks in a specific wavelength where light is absorbed. In addition, concentrations of the odor-causing substances may be analyzed using the absorbed light level (that is, absorbance).

The absorbance may be measured in a wavelength range of ultraviolet and visible light. In some embodiments, the method may comprise exposing the liquid phase stream to light of wavelengths in the ultraviolet and visible light ranges prior to measurement. The absorbance is preferably measured in the wavelength range of visible light and more preferably measured in the wavelength range of 170 nm to 280 nm. In particular, the absorbance is measured in a specific wavelength range so that all major odorous substances designated by the Korean Ministry can be analyzed. In addition, the analysis efficiency can be improved because analysis in all ultraviolet regions is unnecessary.

In one embodiment, the method further comprises analyzing the odor-causing substances based on the measured absorbance as described above. Through the analysis, analyzing the types and concentrations of the odor-causing substances may be performed. Each of the odor-causing substances may have an inherent absorbance peak in the wavelength range described above. Depending on the absorbance peaks, the types of odor-causing substances may be specified. In addition, the area under the absorbance curve of a specific substance in the absorbance graph is proportional to the concentration of the corresponding substance. Hence, the types and concentrations of the odor-causing substances in the liquid-phase stream may be analyzed by comparing the absorbance data of a specific substance registered in a known database and measured absorbance data.

In the method of the invention, the analysis of the remaining odor-causing substances is performed on the flue gas containing the reduced number of the odor-causing substances after passing through the odor removal unit. Except for quite exceptional cases, such as air pollution caused by accidents of unknown causes, fires, natural disasters, and the like, the main composition of the flue gas generated from a particular emission source is predictable by reactions performed in the emission source, reactants, products, by-products, and the like. In such conditions, analyzing the composition of the odor-causing substances in flue gas upstream of the odor removal unit is ineffective. In the method of the invention, the number of odor-causing substances in the flue gas is reduced by allowing the flue gas to pass through the odor removal unit. The odor-causing substances remaining in the flue gas are analyzed. The operating conditions may be adapted according to the remaining amount of the predictable odor-causing substances. Alternatively or additionally, an additional process may be performed if un-predictable odor-causing substances are present.

Absorbance measurement methods allow monitoring not only the analysis of a single odor-causing substance but also the simultaneous monitoring of a plurality of odor-causing substances. Usually, however, as the number of the odor-causing substances increases, the accuracy and speed of the analysis would decrease. Thus, according to the invention, the measurement is performed in downstream processes of the odor removal unit, so as to improve accuracy and speed of the analysis by reducing the number of the odor-causing substances to be analyzed.

Additionally, in the method of the invention, the analysis of the odor-causing substances is performed in a liquid state, not a gas state, in which the flue gas is dissolved in the solvent. Compared to measuring the absorbance directly on the gas-phase stream discharged from the odor removal unit, measuring the absorbance in the liquid-phase stream by dissolving the portion of the gas-phase stream has the following advantages:
1) in the case of measuring the absorbance of the gas-phase stream, there may be a problem in that the absorbance itself fails to be measured due to the remaining substances in the flue gas, other than water vapor or the odor-causing substances;
2) in the case of measuring the absorbance of the liquid-phase stream, substances that cause measurement errors by interfering with light transmission may be removed, and the odor-causing substances may be concentrated, so the reliability of the measurement and analysis can be improved;
3) in the case of measuring the absorbance of the liquid-phase stream, environmental conditions, such as pH, temperature, and the like, can be uniformly controlled with ease; and
4) in the case of measuring the absorbance of the liquid-phase stream, experimental equipment, and the like can be washed with ease after the measurement, compared to the case when using the gas-phase stream.

In the method of the present invention, the odor-causing substances may be identified and/or quantified based on the measured absorbance as described, and the operating conditions in the odor removal unit then may be controlled based on the analysis results. Alternatively, an additional process may be performed on the gas-phase stream in the downstream processes of the odor-removal unit through a separate unit.

For example, from the measurement results of the absorbance of the liquid-phase stream and the analysis results thereof,
i) when the absorbance of the predictable odor-causing substances is equal to or higher than 90% of the reference value, the operating conditions (water level, flow rate, pH, temperature, and the like) in the odor removal unit may be reinforced. Here, "reference value" may, for example, refer to the upper limit of the concentration of odor-causing substances in flue gas allowed to be discharged into the atmosphere. According to one embodiment, the gas-phase stream is recirculated through the odor removal unit. According to another embodiment, when the odor removal unit is the scrubber, the water stream discharged from the scrubber is recirculated as water supplied to the scrubber through the top of the scrubber to increase a liquid-to-gas ratio in the odor removal unit.
ii) When the absorbance of the predictable odor-causing substances is lower than the reference value by 20% or more, the operating conditions in the odor removal unit may be determined to be extremely intense compared to the current flue gas conditions, so the operating conditions in the odor removal unit may be relaxed.
iii) When the absorbance of unexpected odor-causing substances is higher than the reference value, the gas-phase stream may be introduced into a separate odor removal unit during the downstream processes.

In one embodiment, when using the scrubber as the odor removal unit, the method of the invention further includes measuring the absorbance of the water stream. The water stream contains the odor-causing substances removed from the flue gas. Thus, when the above-measured absorbance and the absorbance measured from the liquid-phase stream are compared and analyzed, the operating conditions in the odor removal unit may be modified for further optimization. In addition, through the analysis of the absorbance measured from the liquid-phase stream, the concentrations of odor-causing substances dissolved in the water stream may be considered to determine whether to recirculate the water stream to the scrubber. This reduces the usage of fresh water required in the scrubber, which is advantageous in terms of costs. Furthermore, in the process in which the scrubber wastewater is constantly recirculated, when measuring the absorbance of the water stream, the concentration of the odor-causing substances in scrubber circulating water can be monitored in real time, and thus can be used as an indicator to determine the time to replace scrubber water, which is advantageous.

### - Sample 1

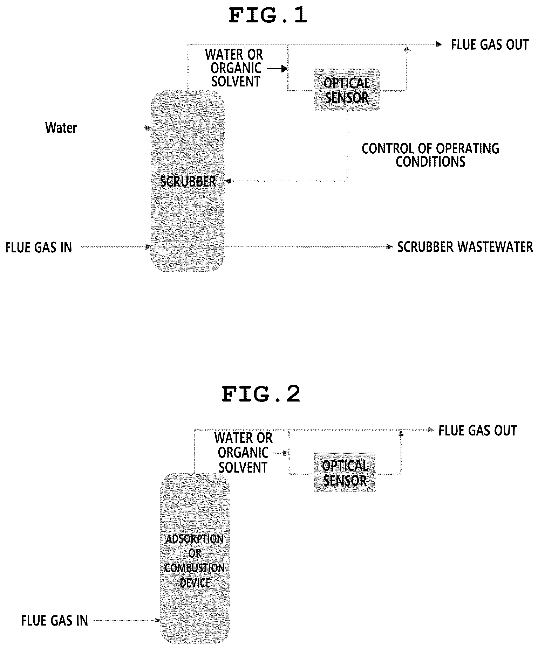

Referring to FIG. 1, disclosed is a flowchart illustrating a process of monitoring odor-causing substances in flue gas according to one embodiment of the invention. When using a scrubber as an odor removal unit, the flue gas is introduced into the bottom of the scrubber, and water is supplied to the top of the scrubber. Then, the water makes contact with the flue gas in the scrubber, through which the odor-causing substances in the flue gas, dissolved in the water, are discharged from the bottom of the scrubber as scrubber wastewater. The rest of the flue gas, after making contact with the water, is discharged from the top of the scrubber. Next, a portion of the flue gas is dissolved in a solvent, and an optical sensor (or spectrophotometer) is used for absorbance measurement. Based on the measured absorbance, operating conditions in the scrubber may be controlled.

### - Sample 2

Referring to FIG. 2, disclosed is a flowchart illustrating a process of monitoring odor-causing substances in flue gas according to one embodiment of the invention. When using an adsorption or combustion device as an odor removal unit, the flue gas is introduced into the bottom of the adsorption or combustion device. The odor-causing substances in the flue gas are adsorbed or combusted in the device, and the rest of the flue gas is discharged from the top of the adsorption or combustion device. Next, a portion of the discharged flue gas is dissolved in a solvent, and an optical sensor (or spectrophotometer) is used for absorbance measurement. Based on the measured absorbance, operating conditions in the adsorption or combustion device can be controlled (not shown).

### - Sample 3

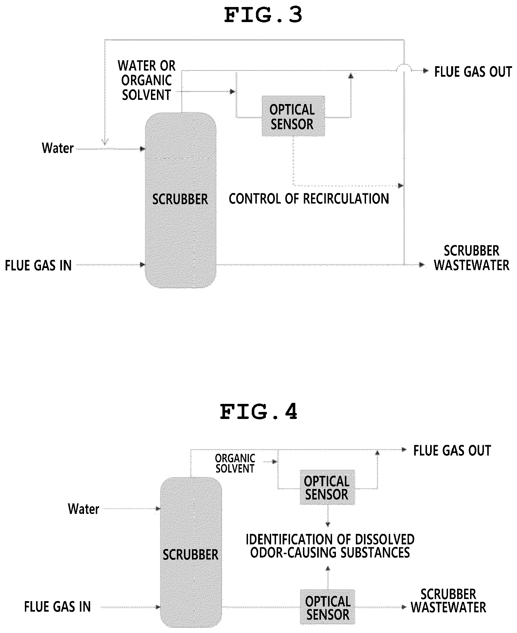

Referring to FIG. 3, disclosed is a flowchart illustrating a process of monitoring odor-causing substances in flue gas according to one embodiment of the invention. When using a scrubber as an odor removal unit, the basic configuration is the same as in Sample 1 described above. Based on the additionally measured absorbance, recirculation of the scrubber wastewater can be controlled.

### - Sample 4

Referring to FIG. 4, disclosed is a flowchart illustrating a process of monitoring odor-causing substances in flue gas according to one embodiment of the invention. When using a scrubber as an odor removal unit, the basic configuration is the same as in Sample 1 described above. An optical sensor (or spectrophotometer) is used for absorbance measurement of the scrubber wastewater discharged from the bottom of the scrubber. With the comprehensive analysis of the absorbance of the scrubber wastewater and the absorbance of the flue gas dissolved in the solvent, operating conditions in the scrubber (not shown), recirculation of the scrubber wastewater, and/or recirculation of the flue gas can be controlled.

### Example

### 1. Measurement of absorbance of odor-causing substances

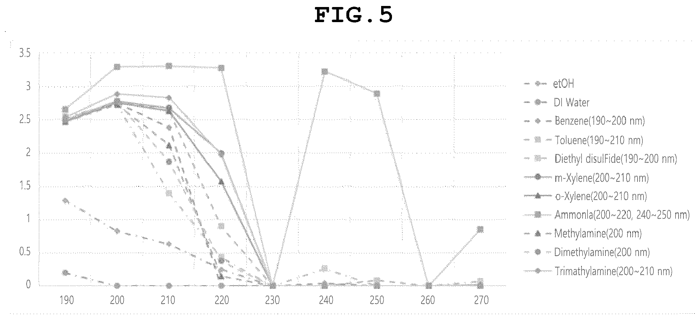

The absorbance of known odor-causing substances was measured. Specific odor-causing substances are shown in Tables 3 and 4 below. A spectrophotometer (Evolution 60S) manufactured by Thermo Fisher Scientific was used for absorbance measurement. Each of the substances specified in Tables 3 and 4 below was dissolved in water at a concentration of 1000 ppm (v/v), and then transferred to a quartz cuvette to measure the absorbance in a wavelength range of 190 nm to 270 nm. Due to solubility limitations, toluene and xylene were dissolved at concentrations of 520 ppm and 200 ppm, respectively, and then measured in the same manner. The absorbance measurement results for each substance are also shown in Tables 3 and 4. In addition, a graph based on the data shown in Table 3 is shown in FIG. 5.

**[Table 3]**

| Wavelength | etOH | DI water | Benzene | Toluene | Dimethyl disulfide | m-Xylene | o-Xylene | Ammonia | Methyl amine | Dimethyl amine | Trimethyl amine |
|---|---|---|---|---|---|---|---|---|---|---|---|
| 190 | 1.283 | 0.2 | 2.508 | 2.496 | 2.54 | 2.494 | 2.464 | 2.65 | 2.468 | 2.488 | 2.533 |
| 200 | 0.821 | 0 | 2.745 | 2.772 | 2.73 | 2.77 | 2.746 | 3.288 | 2.72 | 2.769 | 2.88 |
| 210 | 0.633 | 0 | 2.377 | 2.626 | 139 | 2.669 | 2.626 | 3.302 | 2.115 | 1.861 | 2.823 |
| 220 | 0.25 | 0 | 0 | 0.897 | 0.433 | 1.992 | 1.569 | 3.271 | 0.146 | 0.375 | 1.968 |
| 230 | 0 | 0 | 0 | 0 | 0 | 0 | 0 | 0 | 0 | 0 | 0 |
| 240 | 0 | 0 | 0.038 | 0 | 0.265 | 0 | 0 | 3.215 | 0 | 0 | 0 |
| 250 | 0 | 0 | 0 | 0.062 | 0 | 0 | 0 | 2.888 | 0 | 0 | 0 |
| 260 | 0 | 0 | 0 | 0 | 0 | 0 | 0 | 0 | 0 | 0 | 0 |
| 270 | 0 | 0 | 0 | 0.003 | 0.07 | 0 | 0.015 | 0.852 | 0 | 0 | 0 |

**[Table 4]**

| Chemical Name | λmax (nm) | Chemical Name | λmax (nm) | Chemical Name | λmax (nm) |
|---|---|---|---|---|---|
| SO₂ | 280 to 290 | Trimethylamine | About 190.5 | Phenol | 210 or lower, 265 to 275 |
| SO₂ in water | 270 to 280 | Dimethylamine | About 190.5 | Styrene | 240 to 250 |
| H₂S | 220 or lower | Skatole | About 223 | o-Cresol | 220 or lower, 270 to 280 |
| Methyl mercaptan | 225 to 235 | Ethylamine | About 177,213 | *m*-Cresol | 220 or lower, 270 to 285 |
| Ethyl mercaptan | 215 or lower | Acetaldehyde | 285 to 295 | Xylene | 210 or lower |
| Dimethyl disulfide | 200 or lower | Benzene | 175 to 180,210 or lower | Methyl cyclohexane | About 207 |
| Methyl ethyl sulfide | 205 or lower | Toluene | 255 to 265 | Propionaldehyde | About 282 |
| Ammonia | 190 to 200 | α-Methylstyrene | 215 or lower, 245 to 255 | *n*-Valeraldehyde | About 178, 182,184 |
| Methylamine | 170 to 180, 210 to 220 | Trimethyl benzene | 225 or lower | Ethyl acetate | About 209 |
| Indole | 240 or lower | Benzaldehyde | 250 or lower | Butyric acid | About 208 |
| Pyridine | 240 to 260 | Benzoic acid | 215 to 225 | Ethyl acrylate | About 208 |

As a result of measuring the absorbance of the odor-causing substances, it was seen that each of the odor-causing substances had an inherent absorbance at a specific wavelength in a wavelength band range of about 170 nm to 280 nm as seen from Tables 3 and 4, and FIG. 5. Based on these results, the measurement and analysis of the absorbance in the wavelength band are performed by dissolving flue gas in a solvent, so that types and concentrations of the odor-causing substances can be confirmed. Thus, through a monitoring method as described in the present disclosure, an odor removal system can be controlled with high efficiency at low costs.

## Claims

1. A method of monitoring the presence and preferably the type and/or amount of one or more odor-causing substances in a flue gas, the method comprising:
providing a flue gas comprising one or more odor-causing substances;
introducing the flue gas into an odor removal unit to produce a gas-phase stream;
dissolving a portion of the gas-phase stream in a solvent to produce a liquid-phase stream; and
measuring an absorbance of the liquid-phase stream to detect and preferably identify and/or quantify the one or more odor-causing substances.

2. The method of claim 1, wherein the odor-causing substances comprise one or more of ethanol, benzene, trimethyl benzene, benzaldehyde, benzoic acid, toluene, phenol, styrene, α-methylstyrene, indole, pyridine, skatole, methylcyclohexane, xylene, *o*-cresol, *m*-cresol, methyl ethyl ketone, methyl isobutyl ketone, ammonia, methylamine, ethylamine, dimethylamine, trimethylamine, acetaldehyde, propionaldehyde, butyraldehyde, *n*-valeraldehyde, i-valeraldehyde, ethyl acetate, butyl acetate, propionic acid, *n*-butyric acid, *n*-valeric acid, i-valeric acid, i-butyl alcohol, ethyl acrylate, sulfur dioxide, hydrogen sulfide, dimethyl sulfide, dimethyl disulfide, diethyl disulfide, methyl ethyl sulfide, methyl mercaptan, ethyl mercaptan, carbon disulfide, methyl diethanolamine, diisopropylamine, acrolein, chlorine, or fluorine.

3. The method of claim 1, wherein the flue gas is introduced into the bottom of the odor removal unit, and
the odor removal unit comprises a scrubber, a combustion device, an adsorption device, or a unit comprising a combination of such devices.

4. The method of claim 3, wherein the odor removal unit is a scrubber and water is supplied to the top of the scrubber.

5. The method of claim 1, wherein the solvent comprises one or more of water, an aqueous solution, or an organic solvent.

6. The method of claim 5, wherein the aqueous solution is a NaOH solution or H₂SO₄ solution.

7. The method of claim 1, wherein the absorbance is measured using a spectrophotometer or optical sensor.

8. The method of claim 1, wherein the absorbance is measured in a wavelength range of 170 nm to 280 nm.

9. The method of claim 1, further comprising:
analyzing the odor-causing substance based on the measured absorbance to obtain an analysis result; and
controlling an operating condition in the odor removal unit based on the analysis result.
